# EUROPÄISCHE PATENTSCHRIFT

(11) **EP 4 648 847 B1**
(45) Veröffentlichungstag und Bekanntmachung des Hinweises auf die Patenterteilung: **09.12.2026**
(21) Anmeldenummer: 23841254.8
(22) Anmeldetag: 29.12.2023
(51) Int. Cl.: A61N 5/10

(54) **MULTILAMELLENKOLLIMATOR UND BESTRAHLUNGSSYSTEM**
MULTILEAF COLLIMATOR AND IRRADIATION SYSTEM
COLLIMATEUR MULTILAME ET SYSTÈME D'IRRADIATION

(30) Priorität: 11.01.2023 DE 102023100490
(43) Veröffentlichungstag der Anmeldung: 19.11.2025
(73) Patentinhaber: gKteso MedSolutions GmbH, 90592 Schwarzenbruck (DE)
(72) Erfinder: GIEB, Klaus, 91058 Erlangen (DE); MORTEZA, Mirdar, 90461 Nürnberg (DE); UELTZHÖFFER, Stefan, 92348 Berg b.NM i.d.OPf. (DE)
(74) Vertreter: Hauck Patent- und Rechtsanwälte PartmbB
(86) Internationale Anmeldenummer: PCT/EP2023/087992
(87) Internationale Veröffentlichungsnummer: WO 2024/149618

(56) Entgegenhaltungen:
- CN-A- 111 467 693
- CN-A- 111 714 789
- US-A1- 2011 199 085
- US-A1- 2016 071 623
- US-A1- 2016 325 117
- US-A1- 2016 325 118

## Beschreibung

Die Erfindung betrifft einen Multilamellenkollimator, umfassend eine Vielzahl von nebeneinander angeordneten Lamellen, wobei die Lamellen zum Anpassen der Form eines Bestrahlungsfeldes eines Bestrahlungsgeräts mittels Antriebseinrichtungen translatorisch bewegbar sind, wobei die Antriebseinrichtungen jeweils einen Antriebsmotor und eine auf ein Ende einer Lamelle wirkende, eine Drehbewegung des Antriebsmotors in eine translatorische Bewegung umsetzende Linearantriebseinrichtung umfassen, wobei der Multilamellenkollimator weiterhin eine Messeinrichtung zum Messen der Position der Lamellen umfasst.

Die Erfindung betrifft außerdem ein Bestrahlungssystem, umfassend ein Bestrahlungsgerät, eine Behandlungsliege für einen zu bestrahlenden Patienten, und einen Multilamellenkollimator.

Multilamellenkollimatoren dienen dazu, die Form eines Bestrahlungsfeldes eines Bestrahlungsgeräts insbesondere zur Tumorbehandlung in gewünschter Weise zu formen bzw. anzupassen, so dass genau das zu behandelnde Gewebe eines Patienten bestrahlt wird und möglichst wenig nicht zu behandelndes Gewebe. Dazu ist eine Vielzahl von Lamellen vorgesehen, die dicht nebeneinander angeordnet sind und so ein Lamellenpaket bilden. Zum Anpassen der Form des Bestrahlungsfeldes können die Lamellen individuell translatorisch bewegt werden. Ein Multilamellenkollimator ist beispielsweise bekannt aus WO 2012/027180 A2.

Multilamellenkollimatoren umfassen in der Regel mindestens eine Messeinrichtung zum Messen der Position der Lamellen. Auf dieser Grundlage kann einerseits die richtige Position der Lamellen für die gewünschte Form des Bestrahlungsfeldes überprüft werden. Andererseits kann auf dieser Grundlage die translatorische Bewegung der Lamellen gesteuert werden. Zum Erfassen der Position der Lamellen ist zum Beispiel aus EP 1 815 883 A1 das Messen einer Drehposition eines Antriebsmotors mittels Encodern bzw. Potentiometern bekannt. Die Lamellenposition kann so indirekt über die Drehposition des Antriebsmotors erfasst werden. Zwischen dem Antriebsmotor und der jeweiligen Lamelle auftretende Veränderungen, die sich auf die Position der Lamelle auswirken können, können hingegen nicht erfasst werden. Daher kann ein weiteres Messsystem vorgesehen sein, um durch eine entsprechende Redundanz auch vorgenannte Veränderungen sicher zu erfassen. Das primäre und das sekundäre Messsystem müssen unabhängig voneinander arbeiten, insbesondere um zu vermeiden, dass sich eine einzige Veränderung im Messweg auf die Messergebnisse des primären und des sekundären Messsystems auswirkt (kein Single Mode of Failure).

Aus EP 3 827 882 A1 ist eine optische Positionserfassung der Lamellen bekannt, insbesondere mittels Kamerasystemen, wobei auf den Lamellen aufgebrachte Marker erfasst werden können. Eine Auswertung kann zum Beispiel mit Bildverarbeitungsmethoden erfolgen. Mit diesem Messsystem kann die Position der jeweiligen Lamelle direkt erfasst werden, so dass oben genannte Einflüsse auf Messergebnisse vermieden werden können. Allerdings kann eine Anordnung von optischen Messsystemen, wie Kameras, oder anderweitiger elektromagnetisch empfindlicher Systeme in der Nähe eines Hochenergiestrahlengangs eines Bestrahlungsgeräts zu einer Beeinträchtigung des Messergebnisses und/oder einer Verringerung der Lebensdauer des Messsystems führen, insbesondere durch Streustrahlung. Auch verursacht die Anordnung eines Messsystems zum unmittelbaren Messen der Position der Lamellen zusätzlichen Bauraum, was insbesondere bei zum Erreichen einer hohen Auflösung der Formung des Bestrahlungsfeldes möglichst dünnen und eng beieinander angeordneten Lamellen ein Problem darstellen kann. Außerdem erfordert dieses Messsystem ein Integration in das Bestrahlungsgerät selbst, was eine Nachrüstung in bestehenden Bestrahlungssystemen schwierig macht. Ferner wird auf das Dokument US2016/0325117 A1 verwiesen.

Ausgehend von dem erläuterten Stand der Technik liegt der Erfindung daher die Aufgabe zugrunde, einen Multilamellenkollimator und ein Bestrahlungssystem der eingangs genannten Art bereitzustellen, mit denen die Position der Lamellen in einfacher, robuster und kompakter Weise präzise und zuverlässig ermittelt werden kann.

Die Erfindung löst die Aufgabe durch die Gegenstände der Ansprüche 1 und 16. Vorteilhafte Ausgestaltungen finden sich in den abhängigen Ansprüchen, der Beschreibung und den Figuren.

Für einen Multilamellenkollimator löst die Erfindung die Aufgabe dadurch, dass die Messeinrichtung eine Trägerplatte mit einer Vielzahl von Durchgangsöffnungen aufweist, durch die jeweils eine Linearantriebseinrichtung geführt ist, und dass auf der Trägerplatte eine Vielzahl von Detektoren angeordnet ist, die zum Messen der Position jeweils einer Lamelle eine Drehbewegung der auf das Ende der jeweiligen Lamelle wirkenden Linearantriebseinrichtung messen.

In an sich bekannter Weise umfasst der erfindungsgemäße Multilamellenkollimator eine Vielzahl von Lamellen, die nebeneinander angeordnet sind und auf diese Weise ein Lamellenpaket bilden können. Der Multilamellenkollimator dient zum Anpassen der Form eines Bestrahlungsfeldes eines Bestrahlungsgeräts, insbesondere eines Linearbeschleunigers (LINAC). Mit dem Linearbeschleuniger kann im Rahmen einer Strahlentherapie zur Tumorbehandlung zu bestrahlendes Gewebe eines Patientenkörpers bestrahlt werden. Das Bestrahlungsgerät kann während der Bestrahlung um den auf einer Behandlungsliege angeordneten Patientenkörper rotieren. Auch möglich ist es, dass das Bestrahlungsgerät starr angeordnet ist und die Behandlungsliege bzw. der darauf angeordneten Patientenkörper während der Bestrahlung bewegt wird, zum Beispiel rotiert wird.

Die Lamellen bilden einen Stapel von nebeneinander bzw. je nach Orientierung des Bestrahlungsgeräts aufeinander angeordneten Lamellen. Die Lamellen können parallel oder (geringfügig) divergent zueinander angeordnet sein. Die Lamellen sind dicht nebeneinander angeordnet. Es ist insbesondere möglich, dass zwischen benachbarten Lamellen kein Spalt besteht. Benachbarte Lamellen können sich berühren. Die Lamellen bestehen aus einem Material, das den Behandlungsstrahl des Bestrahlungsgeräts stark abschwächt, zum Beispiel Wolfram. Zum Anpassen der Form des Bestrahlungsfeldes des Bestrahlungsgeräts können die Lamellen einzeln translatorisch bewegt werden, nämlich insbesondere parallel zu ihren Seitenflächen. Die individuelle translatorische Bewegung ist insbesondere entlang (nur) einer Bewegungsachse möglich. Damit wird durch die Lamellen ein Durchgang für den Behandlungsstrahl des Bestrahlungsgeräts wahlweise freigegeben oder blockiert, wodurch die Form bzw. Geometrie des durch das Bestrahlungsgerät auf dem Patientenkörper erzeugten Bestrahlungsfeldes so eingestellt werden kann, dass das zu bestrahlende Gewebe, und nach Möglichkeit nur das zu bestrahlende Gewebe, bestrahlt wird.

Wie bereits erläutert, können die Lamellen ein Lamellenpaket bilden. Das Lamellenpaket kann mindestens 30 Lamellen, bevorzugt mindestens 50 Lamellen, umfassen. Die Lamellen können grundsätzlich eine beliebige Dicke besitzen. Sie können insbesondere eine geringe Dicke besitzen. Zum Beispiel können die Lamellen eine Dicke von weniger als 3 mm, insbesondere weniger als 2 mm, aufweisen. Hierdurch wird eine besonders hohe Auflösung der Formung des Bestrahlungsfeldes ermöglicht. Insbesondere jeder Lamelle ist eine Antriebseinrichtung zugeordnet. Die Antriebseinrichtungen umfassen jeweils einen Antriebsmotor. Bei den Antriebsmotoren kann es sich um Elektromotoren handeln. Weiter umfassen die Antriebseinrichtungen jeweils eine Linearantriebseinrichtung, die eine Drehbewegung des Antriebsmotors in eine translatorische Bewegung der jeweiligen Lamelle umsetzt. Die Linearantriebseinrichtung wirkt dazu auf ein Ende einer Lamelle und überträgt so ihre translatorische Bewegung auf die jeweilige Lamelle. Die Linearantriebseinrichtungen sind somit einerseits jeweils mit einem Antriebsmotor gekoppelt, werden also durch den jeweiligen Antriebsmotor drehend angetrieben. Die Linearantriebseinrichtungen können direkt mit dem jeweiligen Antriebsmotor gekoppelt sein. Andererseits wirken die Linearantriebseinrichtungen auf ein Ende jeweils einer Lamelle ein. Die Linearantriebseinrichtungen können jeweils mit dem Ende einer Lamelle direkt oder indirekt mechanisch gekoppelt sein. Die Antriebseinrichtungen können kaskadenartig angeordnet sein und in unterschiedlicher Höhe an den Lamellen angreifen.

Die Messeinrichtung des erfindungsgemäßen Multilamellenkollimators misst über die Rotation der Linearantriebseinrichtung indirekt die translatorische Position der einzelnen Lamellen. Auf Grundlage der Messung kann die Position der Lamellen und damit die Form des Bestrahlungsfeldes des Bestrahlungsgeräts überwacht und eingestellt werden.

Erfindungsgemäß umfasst die Messeinrichtung eine Trägerplatte mit einer Vielzahl von Durchgangsöffnungen, durch die jeweils eine der Linearantriebseinrichtungen geführt ist. Auf der Trägerplatte ist eine Vielzahl von Detektoren angeordnet. Die Detektoren messen zum Bestimmen der Position einer Lamelle die Drehbewegung der auf das Ende dieser Lamelle wirkenden Linearantriebseinrichtung. In einfacher und bauraumsparender Weise sind die Detektoren gemeinsam mit der Trägerplatte in dem ohnehin vorhandenen oder gegebenenfalls geringfügig erweiterten Bauraum zwischen den Antriebsmotoren und den Enden der jeweiligen Lamelle angeordnet. Dazu kann die Trägerplatte in einfacher Weise mit den Durchgangsöffnungen auf die Linearantriebseinrichtungen aufgeschoben werden. Es wird kein relevanter zusätzlicher Bauraum beansprucht. Durch die Integration der Detektoren auf die Trägerplatte ist die Montage einfach, wobei gleichzeitig eine präzise reproduzierbare Anordnung der Detektoren sichergestellt ist. Gleichzeitig können Veränderungen und damit Einflüsse auf die Position bzw. Bewegung der Lamellen zwischen dem Antriebsmotor und der Linearantriebseinrichtung sicher erkannt werden. Die erfindungsgemäße Messeinrichtung eignet sich daher in besonderer Weise in Kombination mit einer weiteren Messeinrichtung, die zum Beispiel mittels Encodern bzw. Potentiometern die Drehbewegung der Antriebsmotoren erfasst. Ein Single Mode of Failure wird vermieden und die erforderliche Redundanz erreicht. Gegenüber einem von der Außenseite aufbauenden, die Lamellenposition direkt erfassenden System, zum Beispiel einem optischen System, vereinfachen sich der Aufbau und der erforderliche Bauraum verringert sich. Auch sind keine die Lamellenform beeinflussenden Komponenten, zum Beispiel eingefräste Taschen für Potentiometer, erforderlich. Es ergibt sich die Möglichkeit einer sehr kompakten Integration der Messeinrichtung in den Multilamellenkollimator. Gleichzeitig ist die präzise Einstellung der Lamellenposition nicht beeinträchtigt. Damit sind besonders dünne Lamellen problemlos möglich, die für eine hohe Auflösung der Formung des Bestrahlungsfeldes erwünscht sind. Die Messrichtung bildet ein berührungslos arbeitendes System und ist kostengünstig. Im Vergleich zu bestehenden Messeinrichtungen werden weniger Teile benötigt und das System ist auch gegenüber Verschmutzungen sehr robust. Störungen durch die Anordnung in der Nähe des Hochenergiestrahls des Bestrahlungsgeräts oder unerwünschte Einflüsse auf die Lebensdauer der Messeinrichtung durch das Bestrahlungsgerät werden vermieden. Das System ist problemlos integrierbar in bestehende Multilamellenkollimatoren. Die Messeinrichtung ist auf eine im Wesentlichen beliebige Anzahl von Lamellen skalierbar, ohne dass sich hierdurch nennenswerte Einflüsse auf die Kosten oder den erforderlichen Bauraum ergeben. Kompatibilität zu bestehenden Systemen ist gewährleistet. Zwar wird mit dem erfindungsgemäßen System die Lamellenposition nicht direkt gemessen, was gegenüber den oben erläuterten optischen Systemen einen gewissen Nachteil bedeuten kann. Abhängig von dem jeweiligen Anwendungsfall wird dies jedoch durch die genannten Vorteile kompensiert.

Es ist möglich, dass jede Lamelle des erfindungsgemäßen Multilamellenkollimators eine erfindungsgemäße Antriebseinrichtung und/oder eine erfindungsgemäße Messeinrichtung umfasst. Es wäre aber auch denkbar, dass der Multilamellenkollimator weiterhin anders angetriebene Lamellen mit einer anders ausgestalteten Antriebseinrichtung und/oder anders in ihrer Position gemessene und/oder eingestellte Lamellen umfasst, insbesondere mit einer anderen Messeinrichtung. Auch denkbar wäre, dass der Multilamellenkollimator Lamellen umfasst, die nicht translatorisch bewegbar sind.

Nach einer Ausgestaltung kann vorgesehen sein, dass mit den Antriebsmotoren drehende Drehelemente der Linearantriebseinrichtungen jeweils mindestens einen Reflektor aufweisen, und dass auf der Trägerplatte weiterhin eine Vielzahl von Sendern angeordnet ist, wobei die Sender jeweils einem der Detektoren zugeordnet sind und im Betrieb der Messeinrichtung elektromagnetische Strahlung auf die Drehelemente aussenden, wobei die von den Sendern ausgesandte elektromagnetische Strahlung durch die den Sendern jeweils zugeordneten Detektoren empfangen wird, wenn der mindestens eine Reflektor im Zuge der Drehung der Drehelemente zu dem Strahlengang der Sender ausgerichtet ist. Der mindestens eine Reflektor reflektiert die von einem Sender ausgesandte elektromagnetische Strahlung im Zuge der Drehung des jeweiligen Drehelements immer dann, wenn die von dem Sender ausgesandte elektromagnetische Strahlung auf den Reflektor trifft. Die reflektierte elektromagnetische Strahlung wird von dem jeweiligen, dem Sender zugeordneten Detektor empfangen. Die Detektoren sind entsprechend angeordnet und ausgerichtet. Im Zuge einer mehrfachen Drehung der Drehelemente kommt es zu mehrfachen Reflektionen und entsprechend einem mehrfachen Empfang elektromagnetischer Strahlung durch die Detektoren. Aus der Detektionsfrequenz der elektromagnetischen Strahlung kann die Drehgeschwindigkeit bzw. die Drehposition des jeweiligen Drehelements und damit die translatorische Bewegung bzw. Position der zugeordneten Lamelle erfasst werden.

Nach einer weiteren Ausgestaltung kann vorgesehen sein, mit den Antriebsmotoren drehende Drehelemente der Linearantriebseinrichtungen jeweils mindestens eine Durchgangsöffnung aufweisen, und dass auf der Trägerplatte weiterhin eine Vielzahl von Sendern angeordnet ist, wobei die Sender jeweils einem der Detektoren zugeordnet sind und im Betrieb der Messeinrichtung elektromagnetische Strahlung auf die Drehelemente aussenden, wobei die von den Sendern ausgesandte elektromagnetische Strahlung durch die den Sendern jeweils zugeordneten Detektoren empfangen wird, wenn die mindestens eine Durchgangsöffnung im Zuge der Drehung der Drehelemente zu dem Strahlengang der Sender ausgerichtet ist. Bei dieser Ausgestaltung weisen die Drehelemente der Linearantriebseinrichtungen jeweils mindestens eine Durchgangsöffnung auf, die von dem jeweiligen Sender ausgesandte elektromagnetische Strahlung durchlässt, wenn die Durchgangsöffnung im Zuge der Drehbewegung des jeweiligen Drehelements zu dem Strahlengang des jeweiligen Senders ausgerichtet ist. Durch die Durchgangsöffnung gelangte elektromagnetische Strahlung wird von dem dem jeweiligen Sender zugeordneten Detektor empfangen. Wiederum sind die Detektoren entsprechend angeordnet und ausgerichtet. Im Zuge der mehrfachen Drehung der Drehelemente kommt es wiederum zu einem mehrfachen Hindurchtreten elektromagnetischer Strahlung durch die Durchgangsöffnung und einem entsprechend mehrfachen Empfang der elektromagnetischen Strahlung durch die jeweiligen Detektoren. Aus der Detektionsfrequenz der elektromagnetischen Strahlung kann wiederum die Drehgeschwindigkeit bzw. Drehposition des jeweiligen Drehelements und damit die translatorische Bewegung bzw. Position der zugeordneten Lamelle erfasst werden.

Die Drehelemente können jeweils eine Mehrzahl von Reflektoren bzw. eine Mehrzahl von Durchgangsöffnungen aufweisen. Insbesondere können jeweils mehrere Reflektoren bzw. mehrere Durchgangsöffnungen über den Umfang der Drehelemente verteilt angeordnet sein. Durch entsprechende Anordnung und Anzahl der Reflektoren bzw. Durchgangsöffnungen kann die Auflösung der Detektion der Drehbewegung gezielt eingestellt, insbesondere erhöht werden. Die Durchgangsöffnungen können zum Beispiel sternförmig in den Drehelementen ausgebildet sein.

Die Sender können Sender für optische Strahlung sein und die Detektoren können Detektoren für optische Strahlung sein. Bei der optischen Strahlung kann es sich um Licht im sichtbaren Frequenzbereich handeln. Es kann sich aber beispielsweise auch um Infrarotstrahlung handeln. Als Sender kommen zum Beispiel Leuchtdioden infrage und als Detektoren Fotodioden. Es sind aber auch andere optische Sender bzw. Detektoren denkbar, zum Beispiel Laser und Laserdetektoren.

Gemäß einer weiteren Ausgestaltung können mit den Antriebsmotoren drehende Drehelemente der Linearantriebseinrichtungen jeweils mindestens einen magnetischen Abschnitt aufweisen, wobei die Detektoren die magnetischen Abschnitte im Zuge der Drehung der Drehelemente detektieren. Bei dieser Ausgestaltung basiert die Erkennung der Drehbewegung der Drehelemente nicht auf elektromagnetischer Strahlung, sondern auf Magnetismus. Die magnetischen Abschnitte können zum Beispiel durch Permanentmagneten oder magnetisierte Abschnitte gebildet sein. Die Detektoren sind entsprechend Magnetdetektoren. Beispielsweise sind Hallsensoren oder GMR-Sensoren (Giant Magnetoresistance Sensoren) geeignet. Im Zuge der mehrfachen Drehung der Drehelemente erfolgt ein mehrfaches Erfassen der magnetischen Abschnitte durch die Detektoren. Aus der Detektionsfrequenz kann wiederum die Drehgeschwindigkeit und Drehposition der Drehelemente und damit die translatorische Bewegung und Position der jeweiligen Lamelle erfasst werden. Wiederum können die Drehelemente jeweils eine Mehrzahl von magnetischen Abschnitten aufweisen, insbesondere über ihren Umfang verteilt angeordnet. Dadurch kann wiederum die Auflösung der Detektion der Drehbewegung erhöht werden.

Nach einer weiteren besonders praxisgemäßen Ausgestaltung können die Drehelemente mit jeweils einem der Antriebsmotoren gekoppelte Gewindestangen von Spindelantrieben sind, wobei die Gewindestangen im Zuge ihrer Drehung jeweils eine mit einem Ende einer Lamelle gekoppelte Spindel oder Gewindemutter translatorisch antreiben. Ebenso möglich ist es, dass die Drehelemente mit jeweils einem der Antriebsmotoren gekoppelte Spindeln oder Gewindemuttern von Spindelantrieben sind, wobei die Spindeln oder Gewindemuttern im Zuge ihrer Drehung jeweils eine mit einem Ende einer Lamelle gekoppelte Gewindestange translatorisch antreiben. Mit einem solchen Spindelantrieb ist eine präzise Einstellung und Erfassung der Drehbewegung der Drehelemente und damit der translatorischen Bewegung der Lamellen besonders einfach, robust und zuverlässig möglich. Es ist bevorzugt, wenn das durch die Detektoren in seiner Drehbewegung erfasste Drehelement axial stillsteht und das Gegenstück des Spindelantriebs translatorisch antreibt, welches wiederum die jeweilige Lamelle translatorisch bewegt. Denkbar ist aber grundsätzlich auch, dass das durch die Detektoren in seiner Drehbewegung erfasste Drehelement sich einerseits dreht und andererseits auch translatorisch bewegt und dadurch die jeweilige Lamelle translatorisch bewegt. Allerdings müssen dann die von den Detektoren zu erfassenden Marker, zum Beispiel Reflektoren, Durchgangsöffnungen oder magnetische Abschnitte, einerseits sowie die Messbereiche der Detektoren andererseits eine ausreichende Erstreckung in Bewegungsrichtung aufweisen, damit jederzeit die Drehbewegung erfasst werden kann.

Der Multilamellenkollimator kann weiterhin eine Auswerteeinrichtung umfassen, die die Messsignale der Detektoren auswertet, wobei die Auswerteeinrichtung auf Grundlage der Auswertung der Messsignale die Position der Lamellen bestimmt und/oder die Antriebseinrichtungen zum translatorischen Bewegen der Lamellen ansteuert.

Nach einer weiteren Ausgestaltung kann vorgesehen sein, dass jeweils mindestens zwei Detektoren zum Messen der Position einer Lamelle vorgesehen sind, wobei die mindestens zwei Detektoren sich überlappende Messbereiche aufweisen, so dass sie im Zuge der Drehbewegung der auf das Ende der jeweiligen Lamelle wirkenden Linearantriebseinrichtung nacheinander ein Messsignal empfangen. Bei dieser Ausgestaltung sind zum Messen der Position einer Lamelle, und damit zum Messen der Drehbewegung einer auf das Ende der jeweiligen Lamelle wirkenden Linearantriebseinrichtung, jeweils zwei Detektoren vorgesehen. Die Messbereiche der beiden Detektoren unterscheiden sich, weisen aber eine teilweise Überlappung auf. Die Messbereiche liegen insbesondere räumlich überlappend nebeneinander. Durch diese Ausgestaltung ist eine eindeutige Identifizierung der Drehrichtung der jeweiligen Linearantriebseinrichtung, insbesondere ihres Drehelements, möglich. Denn ein entsprechendes, die Drehbewegung des Drehelements anzeigendes Messsignal wird von den mindestens zwei Detektoren zeitversetzt empfangen. Durch den Überlapp der Messbereiche liegen außerdem während der Überlappung Messsignale an beiden Detektoren an. Damit kann zum Beispiel ein kurzzeitiges Anhalten oder ein Wechsel der Drehrichtung sicher von einer durchgehenden Drehung in einer Richtung unterschieden werden. Die Drehrichtung ist folglich eindeutig identifizierbar. Entsprechend kann nach einer weiteren Ausgestaltung vorgesehen sein, dass die Auswerteeinrichtung aus den Messignalen der mindestens zwei Detektoren eine Drehrichtung der Drehbewegung der auf das Ende der jeweiligen Lamelle wirkenden Linearantriebseinrichtung bestimmt.

Die Trägerplatte kann gemäß einer besonders praxisgemäßen weiteren Ausgestaltung eine Leiterplatte sein, also ein Printed Circuit Board (PCB). Auf einer solchen Leiterplatte können die Detektoren und gegebenenfalls die Sender einschließlich der elektrischen Versorgung und Leitungsverbindungen für die empfangenen Messsignale besonders zuverlässig und reproduzierbar angeordnet werden.

Grundsätzlich können sämtliche Linearantriebseinrichtungen sämtlicher Lamellen des Multilamellenkollimators durch die Durchgangsöffnungen einer gemeinsamen Trägerplatte geführt sein. Es ist aber auch denkbar, dass die Messeinrichtung mehrere Trägerplatten mit jeweils einer Vielzahl von Durchgangsöffnungen umfasst, wobei durch die Durchgangsöffnungen jeder der Trägerplatten jeweils eine Linearantriebseinrichtung geführt ist, und dass an jeder der Trägerplatten eine Vielzahl von Detektoren angeordnet ist, die zum Messen der Position der Lamellen jeweils eine Drehbewegung einer Linearantriebseinrichtung messen. Bei dieser Ausgestaltung sind also mehrere separate Trägerplatten vorgesehen, die jeweils eine Gruppe von Linearantriebseinrichtungen der gesamten Linearantriebseinrichtungen in ihren Durchgangsöffnungen aufnehmen.

Einige oder sämtliche der Detektoren können separat voneinander ausgebildet sein. Es ist jedoch auch denkbar, dass mindestens einige Detektoren der Vielzahl von Detektoren durch ein Detektorarray gebildet sind. Mehrere Detektoren können also in einem Detektorarray zusammengefasst sein. Unterschiedliche Bereiche des Arrays bilden dann unterschiedliche Detektoren, die einzeln angesteuert und ausgewertet werden.

Nach einer weiteren Ausgestaltung kann vorgesehenen sein, dass der Multilamellenkollimator weiterhin eine Vielzahl von weiteren nebeneinander angeordneten Lamellen umfasst, wobei die weiteren Lamellen gegenüberliegend zu den (ersten) Lamellen angeordnet sind, so dass die (ersten) Lamellen und die weiteren Lamellen zum Anpassen der Form des Bestrahlungsfeldes des Bestrahlungsgeräts mittels Antriebseinrichtungen einander entgegengesetzt translatorisch bewegbar sind. Die Antriebseinrichtungen der weiteren Lamellen können identisch ausgebildet sein zu den Antriebseinrichtungen der (ersten) Lamellen. Auch den weiteren Lamellen kann eine Messeinrichtung zum Messen der Position der weiteren Lamellen zugeordnet sein. Die Messeinrichtung zum Messen der Position der weiteren Lamellen kann identisch ausgebildet sein zu der Messeinrichtung der (ersten) Lamellen. Die Lamellen und die weiteren Lamellen können aufeinander zu und voneinander wegbewegt werden. Zwischen ihren gegenüberliegenden freien Enden bilden sie den Durchgang zur Formung des Behandlungsstrahls bzw. seines Bestrahlungsfeldes auf dem Patientenkörper.

Die Erfindung löst die Aufgabe außerdem durch ein Bestrahlungssystem, umfassend ein Bestrahlungsgerät, eine Behandlungsliege für einen zu bestrahlenden Patienten, und umfassend einen erfindungsgemäßen Multilamellenkollimator. Bei dem Bestrahlungsgerät kann es sich um einen Linearbeschleuniger (LINAC) zur Strahlentherapie eines auf der Behandlungsliege liegenden Patienten handeln. Die Behandlungsliege kann verstellbar sein, zum Beispiel in der Höhe verstellbar und in verschiedenen Richtungen verschiebbar. Auch ein Verkippen der Behandlungsliege kann möglich sein.

Ausführungsbeispiele der Erfindung werden nachfolgend anhand von Figuren näher erläutert. Es zeigen schematisch:
- Figur 1: ein erfindungsgemäßes Bestrahlungssystem in einer Seitenansicht,
- Figur 2: einen Teil eines erfindungsgemäßen Multilamellenkollimators in einer Seitenansicht,
- Figur 3: eine Schnittansicht entsprechend einer Linie A-A in Figur 2,
- Figur 4: eine ausschnittsweise Schnittansicht entsprechend der Darstellung aus Figur 3 für ein weiteres Ausführungsbeispiel eines erfindungsgemäßen Multilamellenkollimators,
- Figur 5: ein Diagramm mit Messsignalen der in Figur 4 gezeigten Detektoren.

Soweit nichts anderes angegeben ist, bezeichnen in den Figuren gleiche Bezugszeichen gleiche Gegenstände.

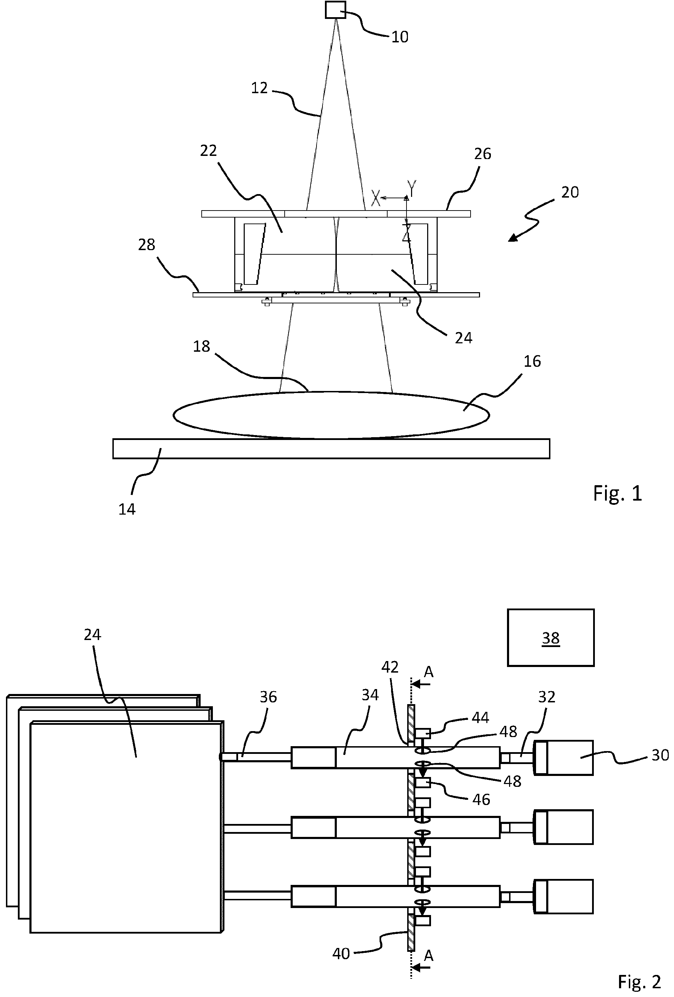

In Figur 1 ist ein Bestrahlungsgerät 10 gezeigt, bei dem es sich insbesondere um einen Linearbeschleuniger (LINAC) zur Tumorbehandlung handelt. Das Bestrahlungsgerät 10 sendet einen Behandlungsstrahl 12 aus, der auf einem auf einer verfahrbaren Behandlungsliege 14 liegenden Patienten 16 ein Bestrahlungsfeld 18 erzeugt. Mittels des Behandlungsstrahls 12 wird zu Therapiezwecken Gewebe insbesondere im Inneren des Körpers des Patienten 16 bestrahlt.

Zwischen dem Bestrahlungsgerät 10 und dem Patienten 16 befindet sich ein Multilamellenkollimator 20, der im gezeigten Beispiel zwei einander gegenüberliegend angeordnete Lamellenpakete umfasst. In den Figuren 1 bis 3 ist ein kartesisches Koordinatensystem zu Veranschaulichungszwecken eingezeichnet. In Figur 1 verläuft die X-Achse von rechts nach links, die Y-Achse in die Zeichenebene hinein und die Z-Achse von oben nach unten. Angetrieben durch nachfolgend näher erläuterten Antriebseinrichtungen können die Lamellen 22, 24 der Lamellenpakete in Figur 1 translatorisch entlang der X-Achse bewegt werden, nämlich aufeinander zu und voneinander weg. Zwischen den einander gegenüberliegenden freien Enden bilden die Lamellen 22, 24 einen Durchgang für die durch das Bestrahlungsgerät 10 ausgesandte Strahlung, wobei durch geeignete Einstellung der translatorischen Position der einzelnen Lamellen 22, 24 das auf dem Körper des Patienten 16 erzeugte Bestrahlungsfeld 18 in gewünschter Weise so in seiner Form angepasst werden kann, dass genau das zu behandelnde Gewebe bestrahlt wird. Die Bewegung der Lamellen 22, 24 wird zwischen Führungswänden 26, 28 geführt.

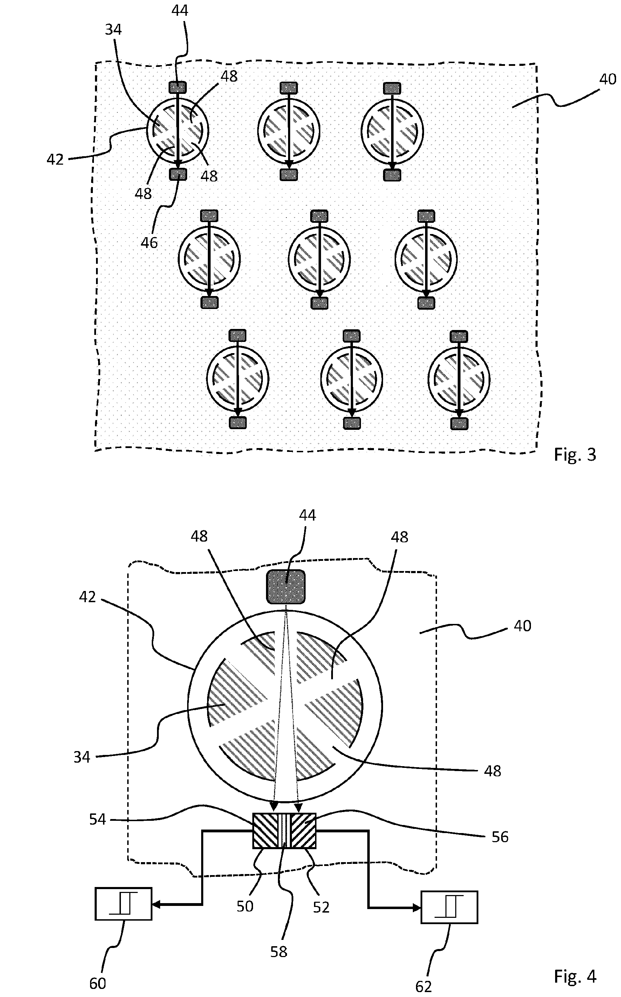

Anhand der Figuren 2 und 3 sollen beispielhaft anhand der in Figur 1 rechten Lamellen 24 die Antriebseinrichtungen der Lamellen 24 und die Messeinrichtung zum Messen der Position der Lamellen 24 näher erläutert werden. Der Antrieb und das Messen der Position der in Figur 1 linken Lamellen 22 kann in identischer Weise erfolgen. Dabei ist in den Figuren 2 und 3 aus Gründen der Übersichtlichkeit nur eine geringe Anzahl von Lamellen 24 mit ihren Linearantriebseinrichtungen dargestellt, wobei in Figur 2 aus Gründen der Übersichtlichkeit weniger Linearantriebseinrichtungen bzw. Lamellen 24 dargestellt sind als in Figur 3. Es versteht sich, dass sämtliche Lamellen 24 Linearantriebseinrichtungen und eine Messeinrichtung aufweisen können, wie nachfolgend beispielhaft erläutert.

Figur 2 zeigt beispielhaft drei Lamellen 24 mit ihren Antriebseinrichtungen. Während Figur 2 eine Seitenansicht ist, sind die Lamellen 24 aus Veranschaulichungsgründen perspektivisch hintereinander dargestellt. Wie in Figur 2 zu erkennen, umfassen die Antriebseinrichtungen für die Lamellen 24 jeweils einen Antriebsmotor 30. Bei den Antriebsmotoren 30 kann es sich um Elektromotoren handeln. Die Antriebsmotoren 30 treiben jeweils über eine Abtriebswelle 32 eine Gewindestange 34 eines Spindelantriebs drehend an. Die Gewindestangen 34 treiben im Zuge ihrer Drehung jeweils eine mit einem Ende einer Lamelle 24 mechanisch gekoppelte Spindel 36 des Spindelantriebs an. Auf diese Weise kann durch Ansteuern der Antriebsmotoren 30 die jeweils zugeordnete Lamelle 24 translatorisch vor und zurück bewegt werden. Das Ansteuern der Antriebsmotoren 30 erfolgt durch eine Auswerteeinrichtung 38 des Multilamellenkollimators, die über entsprechende Steuerleitungen mit den Antriebsmotoren 30 verbunden ist.

Wie in Figur 2 zu erkennen, greifen die Spindeln 36 auf unterschiedlicher Höhe an den Lamellen 24 an. Die obere Spindel 36 greift an der in Figur 2 vorderen Lamelle 24 in einem oberen Bereich der in Figur 2 rechten Kante der Lamelle 24 an, die mittlere Spindel 36 greift an der in Figur 2 mittleren Lamelle 24 in einem mittleren Bereich der in Figur 2 rechten Kante der Lamelle 24 an und die untere Spindel 36 greift an der in Figur 2 hinteren Lamelle 24 in einem unteren Bereich der in Figur 2 rechten Kante der Lamelle 24 an. Durch diese unterschiedlichen Angriffshöhen können die Antriebseinrichtungen mit den Spindeln 36 kaskadenartig übereinander angeordnet werden, wie in Figur 3 zu erkennen. Dabei sind in Figur 2 beispielhaft nur die in Figur 3 linken drei, kaskadenartig übereinander angeordneten Antriebseinrichtungen gezeigt. Durch die kaskadenartige Anordnung können die Antriebseinrichtungen auch an dünnen, eng benachbarten Lamellen angreifen, auch wenn die Antriebseinrichtungen einen größeren Querschnitt besitzen als die Dicke der Lamellen 24.

Die Messeinrichtung des Multilamellenkollimators zum Messen der Position der Lamellen 24 umfasst eine Trägerplatte 40, insbesondere eine Leiterplatte 40 (PCB) mit einer Vielzahl von Durchgangsöffnungen 42, durch die jeweils eine der Gewindestangen 34 der Linearantriebseinrichtungen geführt ist. In Figur 2 ist die Leiterplatte 40 aus Veranschaulichungsgründen geschnitten dargestellt. Auf der Leiterplatte 40 ist eine Vielzahl von einander paarweise gegenüberliegenden Sendern 44 und Detektoren 46 angeordnet. Bei den Sendern 44 und Detektoren 46 handelt es sich um optische Sender 44 bzw. Detektoren 46, zum Beispiel um Leuchtdioden 44 als Sender 44 und Fotodioden 46 als Detektoren 46. Wie in den Figuren 2 und 3 zu erkennen, sind jeder Gewindestange 34 ein Sender 44 und ein Detektor 46 zugeordnet, die jeweils auf einander gegenüberliegenden Seiten der Gewindestangen 34 angeordnet sind. Die Gewindestangen 34 weisen im dargestellten Beispiel jeweils drei sternförmig ausgebildete Durchgangsöffnungen 48 auf. Im Betrieb wird durch die Sender 44 elektromagnetische Strahlung, zum Beispiel Licht im sichtbaren oder nicht sichtbaren Frequenzbereich, ausgesandt, wie in den Figuren 2 und 3 durch Pfeile veranschaulicht. Im Zuge der Drehung der Gewindestangen 34 gelangen die Durchgangsöffnungen 48 nacheinander und mehrfach in Ausrichtung zu dem jeweiligen Strahlengang der Sender 44. Immer wenn eine Durchgangsöffnung 48 im Zuge der Drehung der Gewindestangen 34 mit dem Strahlengang eines Senders 44 ausgerichtet ist, gelangt die von dem Sender 44 ausgesandte Strahlung durch die Durchgangsöffnung 48 hindurch zu dem auf der gegenüberliegenden Seite angeordneten Detektor 46, der diese als Messsignal registriert. Die Messsignale der Detektoren 46 liegen an der Auswerteeinrichtung 38 an. Die Auswerteeinrichtung 38 kann auch die Sender 44 über geeignete Steuerleitungen ansteuern. Im Zuge der fortgesetzten Drehung der Gewindestangen 34 wird somit mit einer bei gegebener Anordnung der Durchgangsöffnung 48 von der Drehgeschwindigkeit der Gewindestangen 34 abhängigen Frequenz ein Messsignal durch die Detektoren 46 detektiert. Auf dieser Grundlage kann durch die Auswerteeinrichtung 38 die Drehbewegung der Gewindestange 34 und damit die translatorische Bewegung der Spindel 36 und mit ihr der jeweiligen Lamelle 24 gemessen werden. Auf Grundlage der Messergebnisse kann die Position der Lamellen 24 durch die Auswerteeinrichtung 38 gemessen und durch Ansteuern der Antriebsmotoren 30 in gewünschter Weise eingestellt werden.

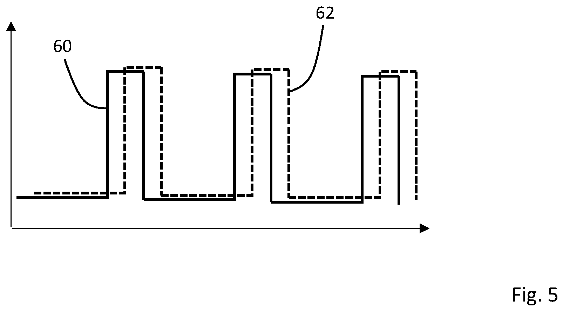

In Figur 4 ist ein abgewandeltes Ausführungsbeispiel der erfindungsgemäßen Messeinrichtung dargestellt. Die in Figur 4 dargestellte Messeinrichtung unterscheidet sich von der in den Figuren 2 und 3 dargestellten Messeinrichtung lediglich darin, dass jedem Sender 44 zwei Detektoren 50, 52 auf einer gegenüberliegenden Seite der Gewindestange 34 zugeordnet sind, deren Messbereiche 54, 56 nebeneinander liegen und eine teilweise Überlappung 58 aufweisen. Wie in Figur 4 zu erkennen, trifft die von dem Sender 44 ausgesandte elektromagnetische Strahlung durch eine Durchgangsöffnung 48 im Zuge einer Drehung der Gewindestange 34 zum Beispiel entgegen dem Uhrzeigersinn zunächst auf den Messbereich 54 des in Figur 4 linken Detektors 50, im Zuge der weiteren Drehbewegung auf die Überlappung 58 der Messbereiche 54, 56 und nachfolgend auf den Messbereich 56 des in Figur 4 rechten Detektors 52. Die entsprechenden Messsignale 60, 62 sind in Figur 4 schematisch dargestellt und in Figur 5 in einem gemeinsamen Diagramm veranschaulicht. Dabei ist auf der Y-Achse die Intensität des von den Detektoren 50, 52 empfangenen Messsignals über der Zeit auf der X-Achse aufgetragen. Das Messsignal 60 des in Figur 4 linken Detektors 50 ist in Figur 5 als durchgezogene Linie dargestellt und das Messsignal 62 des in Figur 4 rechten Detektors 52 als gestrichelte Linie. Zur Veranschaulichung sind die Messsignale 60, 62 jeweils als Rechtecksignale dargestellt, wobei in Figur 5 jeder der Rechteckpeaks einer Durchgangsöffnung 48 der Gewindestange 34 entspricht. In Figur 5 ist der zeitliche Versatz und der zeitliche Überlapp der Messsignale 60, 62 gut zu erkennen. Auf dieser Grundlage lässt sich durch die die Messsignale 60, 62 empfangende Auswerteeinrichtung 38 die Drehrichtung der Antriebsmotoren 30 bzw. der Gewindestangen 34 und damit die translatorische Bewegungsrichtung der Spindel 36 und der Lamellen 24 eindeutig identifizieren.

Die von den Detektoren 46, 50, 52 empfangenen Messsignale können vor der Auswertung durch die Auswerteeinrichtung 38 verstärkt werden und digitalisiert werden.

Die Erfindung wurde anhand der Zeichnungen für ein Ausführungsbeispiel erläutert, bei dem die Gewindestangen 34 als Drehelemente 34 Durchgangsöffnungen 48 aufweisen. Wie erläutert, sind auch andere Möglichkeiten zur Detektion der Drehbewegung von Drehelementen 34 denkbar, beispielsweise das Anordnen von Reflektoren oder magnetischen Abschnitten auf den Drehelementen 34. Bei Einsatz von magnetischen Abschnitten kann gegebenenfalls auf die Sender 44 verzichtet werden. Eine Detektion der magnetischen Abschnitte im Zuge der Drehbewegung der Drehelemente 34 ist dann allein aufgrund geeigneter Magnetdetektoren möglich. Bei Einsatz von Reflektoren auf den Drehelementen 34 müssen die Sender 44 und Empfänger 46, 50, 52 naturgemäß so angeordnet werden, dass von den Sendern 44 ausgesandte und von den Reflektoren reflektierte elektromagnetische Strahlung durch die Detektoren 46, 50, 52 empfangen werden kann.

Es sei weiterhin darauf hingewiesen, dass der erfindungsgemäße Multilamellenkollimator eine weitere Messeinrichtung zum Messen der Position der Lamellen umfassen kann, beispielsweise umfassend Encoder bzw. Potentiometer, mit denen zum indirekten Messen der Position der Lamellen die Drehbewegung der Antriebsmotoren gemessen wird.

### Bezugszeichenliste

- 10: Bestrahlungsgerät
- 12: Behandlungsstrahl
- 14: Behandlungsliege
- 16: Patient
- 18: Bestrahlungsfeld
- 20: Multilamellenkollimator
- 22: Lamellen
- 24: Lamellen
- 26: Führungswand
- 28: Führungswand
- 30: Antriebsmotoren
- 32: Abtriebswelle
- 34: Gewindestangen
- 36: Spindeln
- 38: Auswerteeinrichtung
- 40: Trägerplatte
- 42: Durchgangsöffnungen
- 44: Sender
- 46: Detektoren
- 48: Durchgangsöffnungen
- 50: Detektoren
- 52: Detektoren
- 54: Messbereich
- 56: Messbereich
- 58: Überlappung
- 60: Messsignal
- 62: Messsignal

## Patentansprüche

1. Multilamellenkollimator (10), umfassend eine Vielzahl von nebeneinander angeordneten Lamellen (22, 24), wobei die Lamellen (22, 24) zum Anpassen der Form eines Bestrahlungsfeldes (18) eines Bestrahlungsgeräts (10) mittels Antriebseinrichtungen translatorisch bewegbar sind, wobei die Antriebseinrichtungen jeweils einen Antriebsmotor (30) und eine auf ein Ende einer Lamelle (22, 24) wirkende, eine Drehbewegung des Antriebsmotors (30) in eine translatorische Bewegung umsetzende Linearantriebseinrichtung umfassen, wobei der Multilamellenkollimator (20) weiterhin eine Messeinrichtung zum Messen der Position der Lamellen (22, 24) umfasst, **dadurch gekennzeichnet, dass** die Messeinrichtung eine Trägerplatte (40) mit einer Vielzahl von Durchgangsöffnungen (42) aufweist, durch die jeweils eine Linearantriebseinrichtung geführt ist, und dass auf der Trägerplatte (40) eine Vielzahl von Detektoren (46, 50, 52) angeordnet ist, die zum Messen der Position jeweils einer Lamelle (22, 24) eine Drehbewegung der auf das Ende der jeweiligen Lamelle (22, 24) wirkenden Linearantriebseinrichtung messen.

2. Multilamellenkollimator nach Anspruch 1, **dadurch gekennzeichnet, dass** mit den Antriebsmotoren (30) drehende Drehelemente (34) der Linearantriebseinrichtungen jeweils mindestens einen Reflektor aufweisen, und dass auf der Trägerplatte (40) weiterhin eine Vielzahl von Sendern (44) angeordnet ist, wobei die Sender (44) jeweils einem der Detektoren (46, 50, 52) zugeordnet sind und im Betrieb der Messeinrichtung elektromagnetische Strahlung auf die Drehelemente (34) aussenden, wobei die von den Sendern (44) ausgesandte elektromagnetische Strahlung durch die den Sendern (44) jeweils zugeordneten Detektoren (46, 50, 52) empfangen wird, wenn der mindestens eine Reflektor im Zuge der Drehung der Drehelemente (34) zu dem Strahlengang der Sender (44) ausgerichtet ist.

3. Multilamellenkollimator nach Anspruch 1, **dadurch gekennzeichnet, dass** mit den Antriebsmotoren (30) drehende Drehelemente (34) der Linearantriebseinrichtungen jeweils mindestens eine Durchgangsöffnung (48) aufweisen, und dass auf der Trägerplatte (40) weiterhin eine Vielzahl von Sendern (44) angeordnet ist, wobei die Sender (44) jeweils einem der Detektoren (46, 50, 52) zugeordnet sind und im Betrieb der Messeinrichtung elektromagnetische Strahlung auf die Drehelemente (34) aussenden, wobei die von den Sendern (44) ausgesandte elektromagnetische Strahlung durch die den Sendern (44) jeweils zugeordneten Detektoren (46, 50, 52) empfangen wird, wenn die mindestens eine Durchgangsöffnung (48) im Zuge der Drehung der Drehelemente (34) zu dem Strahlengang der Sender (44) ausgerichtet ist.

4. Multilamellenkollimator nach einem der Ansprüche 2 oder 3, **dadurch gekennzeichnet, dass** die Drehelemente (34) jeweils eine Mehrzahl von Reflektoren oder eine Mehrzahl von Durchgangsöffnungen (48) aufweisen.

5. Multilamellenkollimator nach einem der Ansprüche 2 bis 4, **dadurch gekennzeichnet, dass** die Sender (44) Sender (44) für optische Strahlung sind, und dass die Detektoren (46, 50, 52) Detektoren (46, 50, 52) für optische Strahlung sind.

6. Multilamellenkollimator nach Anspruch 1, **dadurch gekennzeichnet, dass** mit den Antriebsmotoren (30) drehende Drehelemente (34) der Linearantriebseinrichtungen jeweils mindestens einen magnetischen Abschnitt aufweisen, und dass die Detektoren (46, 50, 52) die magnetischen Abschnitte im Zuge der Drehung der Drehelemente (34) detektieren.

7. Multilamellenkollimator nach Anspruch 6, **dadurch gekennzeichnet, dass** die Drehelemente (34) jeweils eine Mehrzahl von magnetischen Abschnitten aufweisen.

8. Multilamellenkollimator nach einem der Ansprüche 2 bis 7, **dadurch gekennzeichnet, dass** die Drehelemente (34) mit jeweils einem der Antriebsmotoren (30) gekoppelte Gewindestangen (34) von Spindelantrieben sind, wobei die Gewindestangen (34) im Zuge ihrer Drehung jeweils eine mit einem Ende einer Lamelle (22, 24) gekoppelte Spindel (36) oder Gewindemutter translatorisch antreiben, oder dass die Drehelemente (34) mit jeweils einem der Antriebsmotoren (30) gekoppelte Spindeln oder Gewindemuttern von Spindelantrieben sind, wobei die Spindeln oder Gewindemuttern im Zuge ihrer Drehung jeweils eine mit einem Ende einer Lamelle (22, 24) gekoppelte Gewindestange translatorisch antreiben.

9. Multilamellenkollimator nach einem der vorhergehenden Ansprüche, **dadurch gekennzeichnet, dass** der Multilamellenkollimator (20) weiterhin eine Auswerteeinrichtung (38) umfasst, die die Messsignale (60, 62) der Detektoren (46, 50, 52) auswertet, wobei die Auswerteeinrichtung (38) auf Grundlage der Auswertung der Messsignale (60, 62) die Position der Lamellen (22, 24) bestimmt und/oder die Antriebseinrichtungen zum translatorischen Bewegen der Lamellen (22, 24) ansteuert.

10. Multilamellenkollimator nach einem der vorhergehenden Ansprüche, **dadurch gekennzeichnet, dass** jeweils mindestens zwei Detektoren (50, 52) zum Messen der Position einer Lamelle (22, 24) vorgesehen sind, wobei die mindestens zwei Detektoren (50, 52) sich überlappende Messbereiche (54, 56, 58) aufweisen, so dass sie im Zuge der Drehbewegung der auf das Ende der jeweiligen Lamelle (22, 24) wirkenden Linearantriebseinrichtung nacheinander ein Messsignal (60, 62) empfangen.

11. Multilamellenkollimator nach den Ansprüchen 9 und 10, **dadurch gekennzeichnet, dass** die Auswerteeinrichtung (38) aus den Messignalen der mindestens zwei Detektoren (50, 52) eine Drehrichtung der Drehbewegung der auf das Ende der jeweiligen Lamelle (22, 24) wirkenden Linearantriebseinrichtung bestimmt.

12. Multilamellenkollimator nach einem der vorhergehenden Ansprüche, **dadurch gekennzeichnet, dass** die Trägerplatte (40) eine Leiterplatte (40) ist.

13. Multilamellenkollimator nach einem der vorhergehenden Ansprüche, **dadurch gekennzeichnet, dass** die Messeinrichtung mehrere Trägerplatten (40) mit einer Vielzahl von Durchgangsöffnungen (42) umfasst, wobei durch die Durchgangsöffnungen (42) jeder der Trägerplatten (40) jeweils eine Linearantriebseinrichtung geführt ist, und dass an jeder der Trägerplatten (40) eine Vielzahl von Detektoren (46, 50, 52) angeordnet ist, die zum Messen der Position der Lamellen (22, 24) jeweils eine Drehbewegung einer Linearantriebseinrichtung messen.

14. Multilamellenkollimator nach einem der vorhergehenden Ansprüche, **dadurch gekennzeichnet, dass** mindestens einige Detektoren (46, 50, 52) der Vielzahl Detektoren (46, 50, 52) durch ein Detektorarray gebildet sind.

15. Multilamellenkollimator nach einem der vorhergehenden Ansprüche, **dadurch gekennzeichnet, dass** er weiterhin eine Vielzahl von weiteren nebeneinander angeordneten Lamellen (22, 24) umfasst, wobei die weiteren Lamellen (22, 24) gegenüberliegend zu den Lamellen (22, 24) angeordnet sind, so dass die Lamellen (22, 24) und die weiteren Lamellen (22, 24) zum Anpassen der Form des Bestrahlungsfeldes (18) des Bestrahlungsgeräts (10) mittels Antriebseinrichtungen einander entgegengesetzt translatorisch bewegbar sind.

16. Bestrahlungssystem, umfassend ein Bestrahlungsgerät (10), eine Behandlungsliege (14) für einen zu bestrahlenden Patienten (16), und umfassend einen Multilamellenkollimator (20) nach einem der vorhergehenden Ansprüche.

## Claims

1. A multileaf collimator (10) comprising a plurality of leaves (22, 24) arranged next to one another, wherein the leaves (22, 24) are translationally movable by means of drive devices for adapting the shape of an irradiation field (18) of an irradiation device (10), wherein the drive devices each comprise a drive motor (30) and a linear drive device which acts on one end of a leaf (22, 24) and converts a rotational movement of the drive motor (30) into a translational movement, wherein the multileaf collimator (20) further comprises a measuring device for measuring the position of the leaves (22, 24), **characterized in that** the measuring device has a carrier plate (40) with a plurality of through-openings (42), through each of which a linear drive device is guided, and **in that** a plurality of detectors (46, 50, 52) is arranged on the carrier plate (40), which detectors, for measuring the position of a respective leaf (22, 24), measure a rotational movement of the linear drive device acting on the end of the respective leaf (22, 24).

2. The multileaf collimator according to claim 1, **characterized in that** rotary elements (34) of the linear drive devices which rotate with the drive motors (30) each have at least one reflector, and **in that** a plurality of emitters (44) is further arranged on the carrier plate (40), wherein the emitters (44) are each assigned to one of the detectors (46, 50, 52) and, during operation of the measuring device, emit electromagnetic radiation onto the rotary elements (34), wherein the electromagnetic radiation emitted by the emitters (44) is received by the detectors (46, 50, 52) respectively assigned to the emitters (44) when the at least one reflector is aligned with the beam path of the emitters (44) in the course of the rotation of the rotary elements (34).

3. The multileaf collimator according to claim 1, **characterized in that** rotary elements (34) of the linear drive devices which rotate with the drive motors (30) each have at least one through-opening (48), and **in that** a plurality of emitters (44) is further arranged on the carrier plate (40), wherein the emitters (44) are each assigned to one of the detectors (46, 50, 52) and, during operation of the measuring device, emit electromagnetic radiation onto the rotary elements (34), wherein the electromagnetic radiation emitted by the emitters (44) is received by the detectors (46, 50, 52) respectively assigned to the emitters (44) when the at least one through-opening (48) is aligned with the beam path of the emitters (44) in the course of the rotation of the rotary elements (34).

4. The multileaf collimator according to claim 2 or 3, **characterized in that** the rotary elements (34) each have a plurality of reflectors or a plurality of through-openings (48).

5. The multileaf collimator according to any one of claims 2 to 4, **characterized in that** the emitters (44) are emitters (44) for optical radiation, and **in that** the detectors (46, 50, 52) are detectors (46, 50, 52) for optical radiation.

6. The multileaf collimator according to claim 1, **characterized in that** rotary elements (34) of the linear drive devices which rotate with the drive motors (30) each have at least one magnetic section, and **in that** the detectors (46, 50, 52) detect the magnetic sections in the course of the rotation of the rotary elements (34).

7. The multileaf collimator according to claim 6, **characterized in that** the rotary elements (34) each have a plurality of magnetic sections.

8. The multileaf collimator according to any one of claims 2 to 7, **characterized in that** the rotary elements (34) are threaded rods (34) of spindle drives, each coupled to one of the drive motors (30), wherein the threaded rods (34), in the course of their rotation, each translationally drive a spindle (36) or threaded nut coupled to one end of a leaf (22, 24), or **in that** the rotary elements (34) are spindles or threaded nuts of spindle drives, each coupled to one of the drive motors (30), wherein the spindles or threaded nuts, in the course of their rotation, each translationally drive a threaded rod coupled to one end of a leaf (22, 24).

9. The multileaf collimator according to any one of the preceding claims, **characterized in that** the multileaf collimator (20) further comprises an evaluation device (38) which evaluates the measurement signals (60, 62) of the detectors (46, 50, 52), wherein the evaluation device (38), on the basis of the evaluation of the measurement signals (60, 62), determines the position of the leaves (22, 24) and/or controls the drive devices for translationally moving the leaves (22, 24).

10. The multileaf collimator according to any one of the preceding claims, **characterized in that** in each case at least two detectors (50, 52) are provided for measuring the position of a leaf (22, 24), wherein the at least two detectors (50, 52) have overlapping measuring ranges (54, 56, 58), so that, in the course of the rotational movement of the linear drive device acting on the end of the respective leaf (22, 24), they successively receive a measurement signal (60, 62).

11. The multileaf collimator according to claims 9 and 10, **characterized in that** the evaluation device (38) determines, from the measurement signals of the at least two detectors (50, 52), a direction of rotation of the rotational movement of the linear drive device acting on the end of the respective leaf (22, 24).

12. The multileaf collimator according to any one of the preceding claims, **characterized in that** the carrier plate (40) is a printed circuit board (40).

13. The multileaf collimator according to any one of the preceding claims, **characterized in that** the measuring device comprises a plurality of carrier plates (40) with a plurality of through-openings (42), wherein a linear drive device is guided through each of the through-openings (42) of each of the carrier plates (40), and **in that** a plurality of detectors (46, 50, 52) is arranged on each of the carrier plates (40), which detectors, for measuring the position of the leaves (22, 24), each measure a rotational movement of a linear drive device.

14. The multileaf collimator according to any one of the preceding claims, **characterized in that** at least some detectors (46, 50, 52) of the plurality of detectors (46, 50, 52) are formed by a detector array.

15. The multileaf collimator according to any one of the preceding claims, **characterized in that** it further comprises a plurality of further leaves (22, 24) arranged next to one another, wherein the further leaves (22, 24) are arranged opposite the leaves (22, 24), so that the leaves (22, 24) and the further leaves (22, 24) are translationally movable in opposite directions to one another by means of drive devices for adapting the shape of the irradiation field (18) of the irradiation device (10).

16. An irradiation system comprising an irradiation device (10), a treatment couch (14) for a patient (16) to be irradiated, and comprising a multileaf collimator (20) according to any one of the preceding claims.

## Revendications

1. Collimateur multi-lames (10) comportant une pluralité de lames (22, 24) disposées côté à côte, dans lequel les lames (22, 24) peuvent être déplacées en translation au moyen de dispositifs d'entraînement afin d'adapter la forme d'un champ d'irradiation (18) d'un appareil d'irradiation (10), dans lequel les dispositifs d'entraînement comportent respectivement un moteur d'entraînement (30) et un dispositif d'entraînement linéaire agissant sur une extrémité d'une lame (22, 24) et convertissant un mouvement de rotation du moteur d'entraînement (30) en un mouvement de translation, le collimateur multi-lames (20) comportant en outre un dispositif de mesure destiné à mesurer la position des lames (22, 24), **caractérisé en ce que** le dispositif de mesure présente une plaque de support (40) pourvue d'une pluralité d'ouvertures de passage (42), à travers lesquelles un dispositif d'entraînement linéaire est guidé respectivement, et **en ce qu'**une pluralité de détecteurs (46, 50, 52) sont disposés sur la plaque de support (40), lesquels mesurent un mouvement de rotation du dispositif d'entraînement linéaire agissant sur l'extrémité d'une lame (22, 24) respective afin de mesurer respectivement la position de la lame (22, 24).

2. Collimateur multi-lames selon la revendication 1, **caractérisé en ce que** des éléments rotatifs (34) des dispositifs d'entraînement linéaire, entraînés en rotation par les moteurs d'entraînement (30), présentent respectivement au moins un réflecteur, et **en ce qu'**une pluralité d'émetteurs (44) sont en outre disposés sur la plaque de support (40), les émetteurs (44) étant respectivement attribués à l'un des détecteurs (46, 50, 52) et émettant un rayonnement électromagnétique vers les éléments rotatifs (34) pendant le fonctionnement du dispositif de mesure, le rayonnement électromagnétique émis par les émetteurs (44) étant reçu par les détecteurs (46, 50, 52) respectivement attribués aux émetteurs (44), lorsque l'au moins un réflecteur est orienté vers le trajet du faisceau des émetteurs (44) pendant la rotation des éléments rotatifs (34).

3. Collimateur multi-lames selon la revendication 1, **caractérisé en ce que** des éléments rotatifs (34) des dispositifs d'entraînement linéaire, entraînés en rotation par les moteurs d'entraînement (30), présentent respectivement au moins une ouverture de passage (48), et **en ce qu'**une pluralité d'émetteurs (44) sont en outre disposés sur la plaque de support (40), les émetteurs (44) étant respectivement attribués à l'un des détecteurs (46, 50, 52) et émettant un rayonnement électromagnétique vers les éléments rotatifs (34) pendant le fonctionnement du dispositif de mesure, le rayonnement électromagnétique émis par les émetteurs (44) étant reçu par les détecteurs (46, 50, 52) respectivement attribués aux émetteurs (44) lorsque l'au moins une ouverture de passage (48) est orientée vers le trajet des faisceaux des émetteurs (44) pendant la rotation des éléments rotatifs (34).

4. Collimateur multi-lames selon l'une des revendications 2 ou 3, **caractérisé en ce que** les éléments rotatifs (34) présentent respectivement une pluralité de réflecteurs ou une pluralité d'ouvertures de passage (48).

5. Collimateur multi-lames selon l'une des revendications 2 à 4, **caractérisé en ce que** les émetteurs (44) sont des émetteurs (44) de rayonnement optique, et **en ce que** les détecteurs (46, 50, 52) sont des détecteurs (46, 50, 52) de rayonnement optique.

6. Collimateur multi-lames selon la revendication 1, **caractérisé en ce que** des éléments rotatifs (34) des dispositifs d'entraînement linéaire, entraînés en rotation par les moteurs d'entraînement (30), présentent respectivement au moins une section magnétique, et **en ce que** les détecteurs (46, 50, 52) détectent les sections magnétiques pendant la rotation des éléments rotatifs (34).

7. Collimateur multi-lames selon la revendication 6, **caractérisé en ce que** les éléments rotatifs (34) présentent respectivement une pluralité de sections magnétiques.

8. Collimateur multi-lames selon l'une des revendications 2 à 7, **caractérisé en ce que** les éléments rotatifs (34) sont des tiges filetées (34) de mécanismes d'entraînement à vis, accouplées respectivement à l'un des moteurs d'entraînement (30), les tiges filetées (34) entraînant respectivement en translation, pendant leur rotation, une vis (36) ou un écrou fileté accouplé à une extrémité d'une lame (22, 24), ou **en ce que** les éléments rotatifs (34) sont des vis ou des écrous filetés de mécanismes d'entraînement à vis, accouplés respectivement à l'un des moteurs d'entraînement (30), les vis ou les écrous filetés entraînant respectivement en translation, pendant leur rotation, une tige filetée accouplée à une extrémité d'une lame (22, 24).

9. Collimateur multi-lames selon l'une des revendications précédentes, **caractérisé en ce que** le collimateur multi-lames (20) comporte en outre un dispositif d'évaluation (38) évaluant les signaux de mesure (60, 62) des détecteurs (46, 50, 52), le dispositif d'évaluation (38) déterminant la position des lames (22, 24) et/ou actionnant les dispositifs d'entraînement pour déplacer en translation les lames (22, 24), à partir de l'évaluation des signaux de mesure (60, 62).

10. Collimateur multi-lames selon l'une des revendications précédentes, **caractérisé en ce qu'**il est prévu respectivement au moins deux détecteurs (50, 52) pour mesurer la position d'une lame (22, 24), les au moins deux détecteurs (50, 52) présentant des plages de mesure (54, 56, 58) se chevauchant entre elles, de sorte qu'ils reçoivent successivement un signal de mesure (60, 62) pendant le mouvement de rotation du dispositif d'entraînement linéaire agissant sur l'extrémité de la lame (22, 24) respective.

11. Collimateur multi-lames selon les revendications 9 et 10, **caractérisé en ce que** le dispositif d'évaluation (38) détermine un sens de rotation du mouvement de rotation du dispositif d'entraînement linéaire agissant sur l'extrémité de la lame (22, 24) respective, à partir des signaux de mesure des au moins deux détecteurs (50, 52).

12. Collimateur multi-lames selon l'une des revendications précédentes, **caractérisé en ce que** la plaque de support (40) est une carte de circuit imprimé (40).

13. Collimateur multi-lames selon l'une des revendications précédentes, **caractérisé en ce que** le dispositif de mesure comporte plusieurs plaques de support (40) pourvues d'une pluralité d'ouvertures de passage (42), un dispositif d'entraînement linéaire étant guidé respectivement à travers les ouvertures de passage (42) de chacune des plaques de support (40), et **en ce qu'**une pluralité de détecteurs (46, 50, 52), mesurant respectivement un mouvement de rotation d'un dispositif d'entraînement linéaire pour mesurer la position des lames (22, 24), sont disposés sur chacune des plaques de support (40).

14. Collimateur multi-lames selon l'une des revendications précédentes, **caractérisé en ce qu'**au moins quelques détecteurs (46, 50, 52) parmi la pluralité de détecteurs (46, 50, 52) sont constitués par un réseau de détecteurs.

15. Collimateur multi-lames selon l'une des revendications précédentes, **caractérisé en ce qu'**il comporte en outre une pluralité de lames supplémentaires (22, 24) disposées côte à côte, les lames supplémentaires (22, 24) étant disposées en face des lames (22, 24), de telle façon que les lames (22, 24) et les lames supplémentaires (22, 24) sont déplaçables en translation dans des directions opposées les unes par rapport aux autres au moyen de dispositifs d'entraînement afin d'adapter la forme du champ d'irradiation (18) de l'appareil d'irradiation (10).

16. Système d'irradiation comportant un appareil d'irradiation (10), une table de traitement (14) destinée à un patient (16) à irradier, et comportant un collimateur multi-lames (20) selon l'une des revendications précédentes.
